# EUROPÄISCHE PATENTSCHRIFT

(11) **EP 1 888 503 B1**
(45) Veröffentlichungstag und Bekanntmachung des Hinweises auf die Patenterteilung: **22.02.2012**
(21) Anmeldenummer: 06763134.1
(22) Anmeldetag: 12.05.2006
(51) Int. Cl.: C07C 51/09, C07C 51/377, C07C 67/10, C07C 67/327, C07C 69/54

(54) **VERFAHREN ZUR HERSTELLUNG VON ALKYL(METH)ACRYLATEN**
METHODS FOR PRODUCING ALKYL(METH)ACRYLATES
PROCEDE DE PRODUCTION DE (METH)ACRYLATES D'ALKYLE

(30) Priorität: 20.05.2005 DE 102005023975
(43) Veröffentlichungstag der Anmeldung: 20.02.2008
(73) Patentinhaber: Evonik Röhm GmbH, 64293 Darmstadt (DE)
(72) Erfinder: ACKERMANN, Jochen, 64367 Mühltal (DE); MAY, Alexander, 64289 Darmstadt (DE); GROPP, Udo, 93093 Bad Endorf (DE); SIEGERT, Hermann, 64342 Seeheim-Jugenheim (DE); VOGEL, Bernd, 65197 Wiesbaden (DE); BRÖCKER, Sönke, 64372 Ober-Ramstadt (DE)
(86) Internationale Anmeldenummer: PCT/EP2006/062281
(87) Internationale Veröffentlichungsnummer: WO 2006/122911

(56) Entgegenhaltungen:
- EP-A- 0 429 800

## Beschreibung

Die vorliegende Erfindung betrifft Verfahren zur Herstellung von Alkyl(meth)acrylaten.

Acrylsäureester und Methacrylsäureester, nachfolgend als Alkyl(meth)acrylate bezeichnet, finden ihr Haupteinsatzgebiet in der Herstellung von Polymeren und Copolymeren mit anderen polymerisierbaren Verbindungen.

Methacrylsäureester, wie zum Beispiel Methylmethacrylat, ist zudem ein wichtiger Baustein für diverse, auf Methacrylsäure (MAS) basierender Spezialester, die durch Umesterung mit dem entsprechenden Alkohol hergestellt werden.

Methylmethacrylat (MMA) und Methacrylsäure werden heute überwiegend ausgehend von Blausäure und Aceton über das entstehende Acetoncyanhydrin (ACH) als Zentralintermediat hergestellt.

Weitere Verfahren, die eine andere Rohstoffbasis als ACH verwenden, sind in der einschlägigen Patentliteratur beschrieben und mittlerweile im Produktionsmaßstab realisiert worden. In diesem Zusammenhang werden heute C-4 basierte Rohstoffe wie Isobutylen oder tert-Butanol als Edukte verwendet, die über mehrere Verfahrenstufen in die gewünschten Methacrylsäurederivate umgewandelt werden.

Intensiv untersucht wurde darüber hinaus die Verwendung von Propen als Basisrohstoff, wobei man über die Stufen Hydrocarbonylierung (zur Isobuttersäure) und dehydrierender Oxidation in moderaten Ausbeuten zur Methacrylsäure gelangt.

Es ist bekannt, Propanal oder Propionsäure, die in technischen Prozessen ausgehend von Ethylen und C-1 Bausteinen wie Kohlenmonoxid zugänglich sind, als Basisrohstoff einzusetzen. In diesen Prozessen wird in einer aldolisierenden Reaktion mit Formaldehyd unter Dehydratisierung der *in situ* entstehenden β-Hydroxy-carbonylverbindung zur entsprechenden α, β-ungesättigten Verbindung umgesetzt. Eine Übersicht über die gängigen Verfahren zur Herstellung der Methacrylsäure und deren Ester findet sich in der Literatur wie Weissermel, Arpe "Industrielle organische Chemie", VCH, Weinheim 1994, 4. Auflage, S.305 ff oder Kirk Othmer "Encyclopedia of Chemical Technology", 3. Ausgabe, Vol. 15, Seite 357.

Es ist allgemein bekannt, dass technische, auf ACH basierende Verfahren mit hochkonzentrierter Schwefelsäure (um etwa 100 Gew.-% H₂SO₄) im ersten Schritt der Umsetzung, der sogenannten Amidierung, bei Temperaturen zwischen 80°C bis etwa 110°C durchgeführt werden.

Repräsentativ für einen solchen Prozess ist beispielsweise US Patent 4,529,816, in der die ACH Amidierung bei Temperaturen um 100°C mit einem molaren Verhältnis von ACH : H₂SO₄ von etwa 1 : 1,5 bis 1 : 1,8 durchgeführt wird. Verfahrensrelevante Prozessschritte für diesen Prozess sind: a) Amidierung; b) Konvertierung; und c) Veresterung.

In der Amidierung werden als Hauptprodukte der Umsetzung SIBA = Sulfoxy-alpha-Hydroxyisobuttersäureamid-Hydrogensulfat und MASA x H₂SO₄ = Methacrylsäureamid-Hydrogensulfat als Lösung in überschüssiger Schwefelsäure erhalten. Des Weiteren ist in einer typischen Amidierungslösung noch HIBA x H₂SO₄ = alpha-Hydroxyisobuttersäureamid-Hydrogensulfat mit einer Ausbeute bezüglich ACH von < 5% erhalten. Bei mehr oder weniger vollständigem ACH Umsatz verläuft dieser an sich recht selektive Amidierungsprozess mit einer Ausbeute (= Summe der beschriebenen Intermediate) von ca. 96-97%.

Als Nebenprodukte in diesem Schritt werden damit aber bereits in nicht unerheblichen Mengen Kohlenmonoxid, Aceton, Sulfonierungsprodukte des Acetons und Cyclokondensationsprodukte von Aceton mit diversen Intermediaten gebildet.

Je nach Wassergehalt in der verwendeten Schwefelsäure stellt sich auch der Anteil von HIBA neben SIBA in der Amidierungsmischung ein. Verwendet man beispielsweise eine 97 Gew.-%ige Schwefelsäure (1,5 Äquivalente H₂SO₄ bzgl. ACH), so entstehen bereits um die 25 Gew.-% HIBA, die nicht mehr selektiv und vollständig in der Konvertierung zu MASA umgesetzt werden können. Durch den relativ hohen Wasseranteil in der Amidierung bei Temperaturen von 90°C - 110°C bedingt sich also ein relativ hoher Anteil an HIBA, der durch konventionelle Konvertierung nur relativ unselektiv zum Zielintermediat MASA x H₂SO₄ umgewandelt werden kann.

Ziel der Konvertierung ist die möglichst vollständige Umsetzung von SIBA und HIBA zu MASA, die unter β-Eliminierung von Schwefelsäure (in überschüssiger Schwefelsäure als Lösungsmittel) verläuft.

In dem Verfahrensschritt Konvertierung wird nun die schwefelsaure (wasserfreie) Lösung aus HIBA, SIBA und MASA (liegen jeweils als Hydrogensulfate vor) bei hohen Temperaturen zwischen 140°C - 160°C und kurzen Verweilzeiten von etwa 10 min oder weniger umgesetzt.

Das Konvertierungsgemisch dieser Verfahrensweise ist charakterisiert durch einen hohen Überschuss an Schwefelsäure und das Vorliegen des Hauptprodukts MASA x H₂SO₄ mit einer Konzentration in der Lösung von etwa 30-35 Gew.-% (je nach eingesetztem Schwefelsäureüberschuss).

Bei mehr oder weniger vollständigem SIBA x H₂SO₄ Umsatz verläuft der Konvertierungsschritt mit einer MASA x H₂SO₄ Ausbeute von ca. 94-95%.

Zuzüglich der Verluste in der Amidierung durch die oben beschriebenen Nebenreaktionen stehen damit für die anschließende Veresterung zum als Produkt gewünschten Methylmethacrylat (MMA) nur zwischen 90-92% MASA (bzgl. ACH) zur Verfügung.

Als Nebenprodukte in diesem Verfahrensschritt werden bedingt durch die drastischen Reaktionsbedingungen erhebliche Mengen an Kondensations-und Additionsprodukten der Intermediate miteinander gebildet.

Ziel der Veresterung ist die möglichst vollständige Umsetzung von MASA x H₂SO₄ aus der Konvertierung zu MMA. Die Veresterung verläuft durch Zugabe eines Gemisches bestehend aus Wasser und Methanol zur MASA - Schwefelsäure Lösung und verläuft zumindest teilweise über Methacrylsäure (MAS) als Intermediat. Die Reaktion kann unter Druck oder drucklos betrieben werden.

Üblicherweise wird hierbei durch Verseifung/Veresterung der Konvertierungslösung bei Temperaturen zwischen 90°C - 140°C bei Reaktionszeiten von ein oder mehreren Stunden eine schwefelsaure Lösung von MMA, MAS und gebildetem Ammoniumhydrogensulfat erhalten.

Durch die Reaktionsbedingungen in Anwesenheit freier Schwefelsäure beträgt die Methanolselektivität in diesem Schritt nur etwa 90% oder weniger, wobei Dimethylether durch Kondensation von Methanol als Nebenprodukt gebildet wird.

Bei mehr oder weniger vollständigem MASA x H₂SO₄ Umsatz verläuft die Veresterung mit einer MMA Ausbeute von ca. 98-99% bezüglich eingesetztem MASA (summarische Selektivität von MAS + MMA). Zuzüglich der Verluste in der Amidierung und der Konvertierung durch die oben beschriebenen Nebenreaktionen können damit im Gesamtprozess über alle Stufen MMA Ausbeuten von maximal 90% bezüglich ACH bei optimaler Reaktionsführung erreicht werden.

Neben den schlechten Gesamtausbeuten des oben beschriebenen Prozesses, die insbesondere im Produktionsmaßstab mit dem Anfall erheblicher Mengen an Abfällen und Abgasen verbunden sind, hat dieser Prozess den Nachteil, dass weit überstöchiometrische Mengen an Schwefelsäure eingesetzt werden müssen. Aus der Ammoniumhydrogensulfat- und Schwefelsäure-haltigen Prozesssäure, die in einer Schwefelsäurekontaktanlage regeneriert wird, sondern sich zudem teerartige, feste Kondensationsprodukte ab, die eine einwandfreie Förderung der Prozesssäure behindern und unter erheblichem Aufwand beseitigt werden müssen.

Aufgrund der drastischen Ausbeuteverluste bei dem oben beschriebenen Verfahren aus US Patent 4,529,816, gibt es einige Vorschläge, ACH in Gegenwart von Wasser zu amidieren und hydrolysieren, wobei die Hydroxyfunktion im Molekülverbund zumindest in den ersten Schritten der Umsetzung erhalten bleibt.

Diese Vorschläge zu einer alternativen Amidierung in Gegenwart von Wasser führen je nach dem, ob in Gegenwart von oder ohne Methanol durchgeführt, entweder zur Entstehung von 2-Hydroxyisobuttersäuremethylester (= HIBSM) oder zur Entstehung von 2-Hydroxyisobuttersäure (= HIBS).

2-Hydroxyisobuttersäure ist ein zentrales Intermediat für die Herstellung von Methacrylsäure und hiervon abgeleitete Methacrylsäureester, insbesondere Methylmethacrylat.

Eine weitere Alternative der Herstellung von Estern der 2-Hydroxyisobuttersäure, insbesondere 2-Hydroxyisobuttersäuremethylester, ausgehend von ACH wird in JP Hei- 4-193845 beschrieben. In JP Hei- 4-193845 wird ACH mit 0,8 bis 1,25 Äquivalenten Schwefelsäure in Gegenwart von weniger als 0,8 Äquivalenten Wasser unterhalb 60°C zunächst amidiert und anschließend bei Temperaturen von größer 55°C mit mehr als 1,2 Äquivalenten Alkohol, insbesondere Methanol, zum HIBSM oder entsprechenden Estern umgesetzt. Auf die Anwesenheit von viskositätserniedrigenden Medien, die stabil gegenüber der Reaktionsmatrix sind, wird hier nicht eingegangen.

Die Nachteile und Probleme dieses Verfahrens sind die technische Umsetzung durch außerordentliche Viskositätsbildung am Ende der Reaktion.

Einige Ansätze zur Verwertung und Umwandlung von HIBSM durch Dehydratisierung zu Methylmethacrylat sind in der Patentliteratur beschrieben.

Zum Beispiel wird in der EP 0 429 800 HIBSM oder eine Mischung aus HIBSM und eines entsprechenden alpha- oder beta-Alkoxyesters in der Gasphase, in Gegenwart von Methanol als Co-Feed an einem heterogenen Katalysator bestehend aus einem kristallinen Alumosilikat und einer Misch-Dotierung aus einem Alkalimetallelement einerseits und einem Edelmetall andererseits umgesetzt. Obwohl Umsatz und Selektivität des Katalysators zumindest zu Beginn der Reaktion ganz gut sind, kommt es mit zunehmender Reaktionszeit zu einer recht drastischen Deaktivierung des Katalysators, die mit absinkenden Ausbeuten einhergeht.

Einen ähnlichen Ansatz verfolgt EP 0 941 984, in der die Gasphasendehydratisierung von HIBSM als Teilschritt einer MMA Synthese in Gegenwart eines heterogenen Katalysators bestehend aus einem Alkalimetallsalz der Phosphorsäure auf SiO₂ beschrieben wird. Insgesamt ist aber dieses vielstufige Verfahren kompliziert, erfordert in Teilschritten erhöhte Drücke und damit teures Equipment und liefert nur unbefriedigende Ausbeuten.

Neben den oben dargestellten Arbeiten zur Dehydratisierung von HIBSM und verwandter Ester zu den entsprechenden alpha-beta ungesättigten Methacrylsäureverbindungen in der Gasphase, gibt es auch Vorschläge zur Durchführung der Reaktion in flüssiger Phase.

Die Herstellung von MAS ausgehend von 2-Hydroxyisobuttersäure wird, zum Beispiel in US 3,487,101 beschrieben, wo die Herstellung diverser Methacrylsäurederivate, insbesondere Methacrylsäure und Methacrylsäureester, ausgehend von 2-Hydroxyisobuttersäure in der Flüssigphase, dadurch gekennzeichnet ist, dass die Umsetzung von HIBS zu Methacrylsäure in Gegenwart eines gelösten basischen Katalysators bei hohen Temperaturen zwischen 180°C - 320°C in Gegenwart hochsiedender Ester (z.b. Phtalsäuredimethylester) und inneren Anhydriden (z.B. Phtalsäureanhydrid) durchgeführt wird. Laut Patent werden bei HIBS Umsätzen > 90% MAS-Selektivitäten um 98% erreicht. Über die Langzeitstabilität der flüssigen Katalysatorlösung insbesondere der Erschöpfung des eingesetzten Anhydrids werden keine Angaben gemacht.

In JP 184047/1985 wird weiterhin die Dehydratisierung von HIBSM in Gegenwart hochkonzentrierter Schwefelsäure (90 - 100 Gew.- %) beschrieben. Nachteilig hierbei sind die hohen Aufwandmengen an Schwefelsäure und der Zwangsanfall großer Mengen wässriger Schwefelsäure, die im Verlauf der Reaktion durch die Wasserfreisetzung aus HIBSM gebildet wird. Eine wirtschaftliche Bedeutung erlangt dieses Verfahren aufgrund der Abfallsäuremengen nicht.

DE-OS 1 191367 betrifft die Herstellung von Methacrylsäure ausgehend von 2-Hydroxyisobuttersäure in der Flüssigphase, dadurch gekennzeichnet, dass die Umsetzung von HIBS zu Methacrylsäure in Gegenwart von Polymerisationsinhibitoren (wie z.B. Kupferpulver) und in Gegenwart eines Katalysatorgemischs bestehend aus Metallhalogeniden und Alkalihalogeniden bei hohen Temperaturen zwischen 180 - 220°C durchgeführt wird. Laut Patent werden bei HIBS Umsätzen > 90% MAS-Selektivitäten von > 99% erreicht. Die besten Ergebnisse werden mit Katalysatormischungen aus Zinkbromid und Lithiumbromid erreicht. Es ist allgemein bekannt, dass die Verwendung halogenid-haltiger Katalysatoren bei hohen Temperaturen drastische Forderungen an die zu verwendenden Werkstoffe stellt und diese Probleme bezüglich der im Destillat befindlichen, verschleppten halogenierten Nebenprodukte auch in nachfolgenden Anlagenteilen auftreten.

EP 0 487 853 beschreibt die Herstellung von Methacrylsäure ausgehend von Acetoncyanhydrin, dadurch gekennzeichnet, dass man im ersten Schritt ACH mit Wasser bei moderaten Temperaturen in Gegenwart eines heterogenen Hydrolysekatalysators umsetzt und man im zweiten Schritt 2-Hydroxyisobuttersäureamid mit Methylformiat oder Methanol/Kohlenmonoxid unter Entstehung von Formamid und Hydroxyisobuttersäuremethylester umsetzt, und man im dritten Schritt HIBSM in Gegenwart eines heterogenen Ionenaustauschers mit Wasser zu Hydroxyisobuttersäure verseift, und man im vierten Schritt HIBS dehydratisiert, indem man in flüssiger Phase bei hohen Temperaturen in Gegenwart eines löslichen Alkalisalzes reagieren lässt. Die Methacrylsäure-Herstellung ex HIBS wird bei hohen Umsätzen um 99% mit mehr oder weniger quantitativen Selektivitäten beschrieben. Die Vielzahl der notwendigen Reaktionsschritte und die Notwendigkeit der Zwischenisolierung einzelner Intermediate, insbesondere auch die Durchführung einzelner Prozessschritte bei erhöhtem Druck, machen das Verfahren kompliziert und damit letztlich unwirtschaftlich. Des Weiteren wird zwingend Formamid hergestellt, wobei diese Verbindung vielfach als unerwünschtes Nebenprodukt angesehen werden kann, welches teuer entsorgt werden muss.

DE-OS 1 768 253 beschreibt ein Verfahren zur Herstellung von Methacrylsäure durch Dehydratisierung von alpha-Hydroxyisobuttersäure, dadurch gekennzeichnet, dass man HIBS in flüssiger Phase bei einer Temperatur von wenigstens 160°C in Gegenwart eines Dehydratisierungskatalysators umsetzt, der aus einem Metallsalz von alpha-Hydroxyisobuttersäure besteht. Besonders geeignet sind in diesem Fall die Alkali- und Erdalkalisalze von HIBS, die in einer HIBS-Schmelze durch Umsetzung geeigneter Metallsalze *in situ* hergestellt werden. Laut Patent werden MAS Ausbeuten bis 95% ex HIBS beschrieben, wobei der Feed der kontinuierlichen Verfahrensweise aus HIBS und ca. 1,5 Gew.-% HIBS-Alkalisalz besteht.

RU 89631 betrifft ein Verfahren zur Herstellung von Methacrylsäure ausgehend von 2-Hydroxyisobuttersäure durch Wasserabspaltung in flüssiger Phase, dadurch gekennzeichnet, dass die Reaktion in Abwesenheit eines Katalysators mit einer wässrigen Lösung von HIBS (bis 62 Gew.-% HIBS in Wasser) unter Druck bei hohen Temperaturen 200°C - 240°C durchgeführt wird.
Es ist außerdem bekannt, dass zur Herstellung von 2-Hydroxyisobuttersäure ausgehend von Acetoncyanhydrin (ACH) die Verseifung der Nitrilfunktion in Gegenwart von Mineralsäuren durchgeführt werden kann (siehe J. Brit. Chem. Soc. (1930); Chem. Ber. 72 (1939), 800).

Repräsentativ für einen solchen Prozess ist beispielsweise die japanische Patentveröffentlichung Sho 63-61932, in der ACH in einem zweistufigen Prozess zur 2-Hydroxyisobuttersäure verseift wird. Hierbei wird ACH zunächst in Gegenwart von 0,2 -1,0 mol Wasser und 0,5 - 2 Äquivalenten Schwefelsäure umgesetzt, wobei die entsprechenden Amidsalze gebildet werden. Bereits in diesem Schritt treten bei Verwendung geringer Wasser-und Schwefelsäurekonzentrationen, die zum Erhalt guter Ausbeuten, kurzer Reaktionszeiten und geringer Abfall-Prozesssäuremengen notwendig sind, massive Probleme mit der Rührbarkeit des Amidierungsgemischs durch hohe Viskosität der Reaktionsansätze insbesondere gegen Ende der Reaktionszeit auf.

Erhöht man die molare Wassermenge zur Gewährleistung einer niedrigen Viskosität, so verlangsamt sich die Reaktion drastisch und es treten Nebenreaktionen auf, insbesondere die Fragmentierung von ACH in die Edukte Aceton und Blausäure, die unter den Reaktionsbedingungen zu Folgeprodukten weiterreagieren. Auch bei Erhöhung der Temperatur lässt sich nach den Vorgaben der japanischen Patentveröffentlichung SHO 63-61932 die Viskosität des Reaktionsgemischs zwar beherrschen und die entsprechenden Reaktionsansätze werden durch die sinkende Viskosität zwar rührbar, aber auch hier nehmen bereits bei moderaten Temperaturen die Nebenreaktionen drastisch zu, was sich letztlich in nur mäßigen Ausbeuten äußert (siehe Vergleichsbeispiele).

Arbeitet man bei niedrigen Temperaturen < 50°C, die eine selektive Reaktionsführung gewährleisten würden, so kommt es gegen Ende der Reaktionszeit durch die Erhöhung der Konzentration der unter den Reaktionsbedingungen schwer löslichen Amidsalzen zunächst zur Bildung einer schwer rührbaren Suspension und schließlich zur vollständigen Verfestigung des Reaktionsansatzes.

Im zweiten Schritt der japanischen Patentveröffentlichung SHO 63-61932 wird Wasser zur Amidierungslösung gegeben und bei höheren Temperaturen als der Amidierungstemperatur hydrolisiert, wobei sich aus den nach der Amidierung gebildeten Amidsalzen unter Freisetzung von Ammoniumhydrogensulfat 2-Hydroxyisobuttersäure bildet.

Wesentlich für die Wirtschaftlichkeit eines technischen Prozesses ist neben der selektiven Darstellung des Zielprodukts HIBS in der Reaktion auch die Isolierung aus der Reaktionsmatrix bzw. die Abtrennung von HIBS von der verbleibenden Prozesssäure.

In JP Sho 57-131736, Methode zur Isolierung von alpha-Oxyisobuttersäure (=HIBS), wird diese Problematik behandelt, indem die nach der Reaktion zwischen Acetoncyanhydrin, Schwefelsäure und Wasser durch hydrolytische Spaltung erhaltene, alpha-Hydroxyisobuttersäure und saures Ammoniumhydrogensulfat enthaltende Reaktionslösung mit einem Extraktionsmittel behandelt wird, wobei die 2-Hydroxyisobuttersäure in das Extraktionsmittel übergeht und das saure Ammonsulfat in der wässrigen Phase zurückbleibt.

Nach diesem Verfahren, wird vor der Extraktion die noch freie Schwefelsäure im Reaktionsmedium durch Behandlung mit einem alkalischen Medium neutralisiert, um den Extraktionsgrad von HIBS in die organische Extraktionsphase zu erhöhen. Die notwendige Neutralisation ist mit einem erheblichen Mehraufwand an aminischer oder mineralischer Base verbunden und damit mit erheblichen Abfallmengen an entsprechenden Salzen, die nicht ökologisch und wirtschaftlich entsorgt werden können.

Die Nachteile des JP Sho 57-131736 Verfahrens zur Darstellung von MMA über Methacrylsäureamid-Hydrogensulfat (Reaktionssequenz: Amidierung - Konvertierung - hydrolytische Veresterung) lassen sich folgendermaßen zusammenfassen:
a.) Verwendung hoher molarer Schwefelsäure Überschüsse bezüglich ACH (im technischen Prozess ca. 1,5 - 2 Äquivalente Schwefelsäure pro Äquivalent ACH).
b.) Hohe Ausbeuteverluste im Amidierungsschritt (ca. 3-4%) und im Konvertierungsschritt (ca. 5-6%), was sich letztlich in einer maximalen Methacrylsäureamidsulfat Ausbeute von ca. 91 % äußert.
c.) Große Abfallströme in Form wässriger Schwefelsäure, in der Ammoniumhydrogensulfat und organische Nebenprodukte gelöst sind. Abscheidung undefinierter Teerrückstände aus dieser Prozess-Abfallssäure, die eine Nachbehandlung bzw. aufwendige Entsorgung notwendig machen.

Die Nachteile des JP Sho 57-131736 Verfahrens zur Darstellung von MMA über Hydroxyisobuttersäure als Zentralintermediat (Reaktionssequenz: Amidierung - Hydrolyse HIBS Synthese - MAS Synthese - hydrolytische Veresterung) lassen sich folgendermaßen zusammenfassen:
a.) Verwendung zwar geringer molarer Schwefelsäure Überschüsse bezüglich ACH (nur ca. 1,0 Äquivalente Schwefelsäure pro Äquivalent ACH), aber massive Probleme mit Viskosität und Rührbarkeit des Amidierungsmediums bis zur kompletten Verfestigung der Reaktionsansätze; die vorgeschlagene Verdünnung der Amidierung mit Alkoholen (Methanol) oder diversen Estern führt unter den Reaktionsbedingungen zur unvollständigen ACH Umsetzung, drastischer Zunahme der Nebenreaktionen oder zur chemischen Zersetzung der Verdünner.
b.) Hohe Ausbeuteverluste im Amidierungsschritt (ca. 5-6%) und aufwendige Extraktion mit einem organischen Lösungsmittel unter Entstehung einer Wasser und HIBS enthaltenden Extraktionsmittelphase, die unter hohem energetischen Aufwand zur Isolierung von HIBS destillativ aufbereitet werden muss. Pro kg HIBS werden etwa 2 kg Prozesssäure-Abfall erzeugt, der etwa 34 Gew.-% Wasser neben 66 Gew.-% Ammoniumhydrogensulfat enthält (siehe japanische Offenlegung SHO-57-131736, Beispiel 4). Die Regenerierung einer Abfallsalzlösung mit hohen Wassergehalten in einer Schwefelsäure-Kontaktanlage (=SK Anlage) ist mit einem erheblichen energetischen Aufwand verbunden, die Kapazität einer solchen SK Anlage deutlich limitiert.

Allen diesen Verfahren ist gemeinsam, dass die Isolierung von HIBS aus der Ammoniumhydrogensulfat-haltigen wässrigen Reaktionsmatrix sehr aufwendig ist. Ein zu hoher Wassergehalt in der HIBS-haltigen Extraktionsphase bedingt auch eine Verschleppung von Ammoniumhydrogensulfat in die nachfolgende MAS Stufe, die nicht mehr im technischen Maßstab über einen vertretbaren Zeitraum hinweg kontinuierlich betrieben werden kann. Der hohe energetische Aufwand bei der Regenerierung hochkonzentrierter wasserhaltiger Prozesssäure- wie auch Extraktionsströme machen die vorgeschlagenen Verfahrensweisen zudem unwirtschaftlich und bieten gegenüber dem zwar unselektiven, aber aufgrund der einfachen, wenigen verfahrenstechnischen Operationen zielgerichteten, etablierten Verfahrensweise, keine wirkliche Alternative.

In Anbetracht des Standes der Technik war es nun Aufgabe der vorliegenden Erfindung, Verfahren zur Herstellung von Alkyl(meth)acrylaten zur Verfügung zu stellen, die einfach und kostengünstig durchgeführt werden können.

Eine weitere Aufgabe der Erfindung bestand darin, ein Verfahren zu schaffen, bei dem die Alkyl(meth)acrylate sehr selektiv erhalten werden können.

Darüber hinaus war es mithin Aufgabe der vorliegenden Erfindung, ein Verfahren zur Herstellung von Alkyl(meth)acrylaten zur Verfügung zu stellen, bei dem nur eine geringe Menge an Nebenprodukten erzeugt wird.

Hierbei sollte das Produkt möglichst in hohen Ausbeuten und, insgesamt gesehen, unter geringem Energieverbrauch erhalten werden.

Ein weiteres Ziel der vorliegenden Erfindung bestand darin ein Verfahren zur Herstellung von Alkyl(meth)acrylaten anzugeben, welches besonders einfach und kostengünstig realisiert werden kann.

Gelöst werden diese sowie weitere nicht explizit genannte Aufgaben, die jedoch aus den hierin einleitend diskutierten Zusammenhängen ohne Weiteres ableitbar oder erschließbar sind, durch Verfahren mit allen Merkmalen des Patentanspruchs 1. Zweckmäßige Abwandlungen der erfindungsgemäßen Verfahren werden in den auf Anspruch 1 zurückbezogenen Unteransprüchen unter Schutz gestellt.

Gegenstand der vorliegenden Erfindung sind dementsprechend Verfahren zur Herstellung von Alkyl(meth)acrylaten, umfassend die Schritte Umesterung eines α-Hydroxycarbonsäurealkylesters mit (Meth)acrylsäure, wobei Alkyl(meth)acrylate sowie α-Hydroxycarbonsäure erhalten werden, und Dehydratisierung der α-Hydroxycarbonsäure, wobei (Meth)acrylsäure erhalten wird.

Durch die erfindungsgemäßen Maßnahmen können des Weiteren unter anderem folgende Vorteile erzielt werden:
Das Verfahren vermeidet die Verwendung von Schwefelsäure in hohen Mengen als Reaktant. Dementsprechend fallen beim erfindungsgemäßen Verfahren keine großen Mengen an Ammoniumhydrogensulfat an.

Durch das erfindungsgemäße Verfahren werden die Alkyl(meth)acrylate in hohen Ausbeuten erhalten. Dies gilt insbesondere im Vergleich mit den in EP-A-0941984 beschriebenen Verfahren, bei denen die α-Hydroxycarbonsäurealkylester unmittelbar zu den Alkyl(meth)acrylaten dehydratisiert werden. Überraschend konnte festgestellt werden, dass durch den zusätzlichen Reaktionsschritt der Umesterung des α-Hydroxycarbonsäurealkylesters mit (Meth)acrylsäure insgesamt höhere Selektivitäten erzielt werden.

Hierbei ist die Bildung von Nebenprodukten ungewöhnlich gering. Des Weiteren werden, insbesondere unter Berücksichtigung der hohen Selektivität, hohe Umsätze erzielt.

Das Verfahren der vorliegenden Erfindung weist eine geringe Bildung von Nebenprodukten auf.

Das erfindungsgemäße Verfahren kann kostengünstig, insbesondere bei geringem Energiebedarf durchgeführt werden. Hierbei können die zur Dehydratisierung und Umesterung verwendeten Katalysatoren über einen langen Zeitraum eingesetzt werden, ohne dass die Selektivität oder die Aktivität abnimmt.

Das Verfahren der vorliegenden Erfindung kann großtechnisch durchgeführt werden.

Erfindungsgemäß werden α-Hydroxycarbonsäurealkylester mit (Meth)acrylsäure umgesetzt. Die hierfür einsetzbaren (Meth)acrylsäuren sind an sich bekannt und können kommerziell erhalten werden. Neben Acrylsäure (Propensäure) und Methacrylsäure (2-Methylpropensäure) gehören hierzu insbesondere Derivate, die Substituenten umfassen. Zu den geeigneten Substituenten gehören insbesondere Halogene, wie Chlor, Fluor und Brom sowie Alkylgruppen, die vorzugsweise 1 bis 10, besonders bevorzugt 1 bis 4 Kohlenstoffatome umfassen können. Hierzu gehören unter anderem β-Methylacrylsäure (Butensäure), α, β-Dimethylacrylsäure, β-Ethylacrylsäure, sowie β,β-Dimethylacrylsäure.

Bevorzugt sind Acrylsäure (Propensäure) und Methacrylsäure (2-Methylpropensäure), wobei Methacrylsäure besonders bevorzugt ist.

Die hierfür eingesetzten α-Hydroxycarbonsäurealkylester sind an sich bekannt, wobei der Alkoholrest des Esters vorzugsweise 1 bis 20 Kohlenstoffatome, insbesondere 1 bis 10 Kohlenstoffatome und besonders bevorzugt 1 bis 5 Kohlenstoffatome umfasst. Bevorzugte Alkoholreste leiten sich insbesondere von Methanol, Ethanol, Propanol, Butanol, insbesondere n-Butanol und 2-Methyl-1-propanol, Pentanol, Hexanol und 2-Ethylhexanol ab, wobei Methanol und Ethanol besonders bevorzugt sind.

Der Säurerest der zur Umesterung eingesetzten α-Hydroxycarbonsäurealkylester leitet sich bevorzugt von der (Meth)acrylsäure ab, die durch Dehydratisierung der α-Hydroxycarbonsäure erhalten werden kann. Wird beispielsweise Methacrylsäure eingesetzt, so wird α-Hydroxyisobuttersäureester verwendet. Wird beispielsweise Acrylsäure eingesetzt, so wird bevorzugt α-Hydroxyisopropionsäure eingesetzt.

Bevorzugt eingesetzte α-Hydroxycarbonsäurealkylester sind α-Hydroxypropionsäuremethylester, α-Hydroxypropionsäureethylester, α-Hydroxyisobuttersäuremethylester und α-Hydroxyisobuttersäureethylester.

Derartige α-Hydroxycarbonsäurealkylester werden vielfach und kostengünstig aus den entsprechenden Cyanhydrinen erhalten. Hierbei ist die Reinheit des Cyanhydrins unkritisch. Demzufolge kann gereinigtes oder ungereinigtes Cyanhydrin zur Hydrolysereaktion eingesetzt werden. Dementsprechend können die erfindungsgemäß einzusetzenden α-Hydroxycarbonsäurealkylester aus Ketonen und Aldehyden sowie Blausäure und einem entsprechenden Alkohol hergestellt werden.

In einem ersten Schritt wird die Carbonylverbindung, beispielsweise ein Keton, insbesondere Aceton, oder ein Aldehyd, beispielsweise Acetaldehyd, Propanal, Butanal, mit Blausäure zum entsprechenden Cyanhydrin umgesetzt. Besonders bevorzugt wird hierbei Aceton und /oder Acetaldehyd auf typische Weise unter Verwendung einer geringen Menge an Alkali oder eines Amins als Katalysator umgesetzt.

In einem weiteren Schritt wird das so erhaltene Cyanhydrin mit Wasser zum Hydroxycarbonsäureamid umgesetzt.

Typischerweise wird diese Umsetzung in Gegenwart eines Katalysators durchgeführt. Hierfür geeignet sind insbesondere Manganoxidkatalysatoren, wie diese beispielsweise in EP-A-0945429, EP-A-0561614 sowie EP-A-0545697 beschrieben sind. Hierbei kann das Manganoxid in Form von Mangandioxid eingesetzt werden, welches durch Behandlung von Mangansulfat mit Kaliumpermanganat unter sauren Bedingungen (vgl. Biochem.J., 50 S. 43 (1951) und J.Chem.Soc., 1953, S. 2189, 1953) oder durch elektrolytische Oxidation von Mangansulfat in wässriger Lösung erhalten wird. Im Allgemeinen wird der Katalysator vielfach in Form von Pulver oder Granulat mit einer geeigneten Korngröße eingesetzt. Des Weiteren kann der Katalysator auf einen Träger aufgebracht werden. Hierbei können insbesondere auch sogenannte Slurry-Reaktoren oder Festbett-Reaktoren eingesetzt werden, die unter anderem in EP-A-956 898 beschrieben sind.

Des Weiteren kann die Hydrolysereaktion durch Enzyme katalysiert werden. Zu den geeigneten Enzymen gehören unter anderem Nitrilhydratasen. Diese Reaktion ist beispielhaft in "Screening, Characterization and Application of Cyanide-resistant Nitrile Hydratases" Eng. Life. Sci. 2004, 4, No. 6 beschrieben.

Darüber hinaus kann die Hydrolysereaktion durch Säuren, insbesondere Schwefelsäure katalysiert werden. Dies wird unter anderem in JP Hei 4-193845 dargelegt.

Das Wasser, welches zur Hydrolyse des Cyanhydrins notwendig ist, kann vielfach als Lösungsmittel eingesetzt werden. Vorzugsweise beträgt das molare Verhältnis von Wasser zu Cyanhydrin mindestens 1, besonders bevorzugt liegt das molare Verhältnis von Wasser zu Cyanhydrin im Bereich von 0,5:1-25:1 und ganz besonders bevorzugt im Bereich von 1:1-10:1.

Das zur Hydrolyse eingesetzte Wasser kann einen hohen Reinheitsgrad aufweisen. Allerdings ist diese Eigenschaft nicht zwingend. So kann neben Frischwasser auch Brauchwasser oder Prozesswasser eingesetzt werden, welches mehr oder minder hohe Mengen an Verunreinigungen umfasst. Dementsprechend kann auch recyceltes Wasser zur Hydrolyse verwendet werden.

Des Weiteren können weitere Bestandteile in der Reaktionsmischung zur Hydrolyse des Cyanhydrins vorhanden sein. Hierzu gehören u.a. Aldehyde und Ketone, insbesondere jene, die zur Herstellung des Cyanhydrins eingesetzt wurden. Beispielsweise kann Aceton und / oder Acetaldehyd in der Reaktionsmischung enthalten sein. Dies wird beispielsweise in US 4018829-A beschrieben. Die Reinheit der zugegebenen Aldehyde und/oder Ketone ist im Allgemeinen nicht besonders kritisch. Dementsprechend können diese Stoffe Verunreinigungen, insbesondere Alkohole, beispielsweise Methanol, Wasser und/oder α-Hydroxyisobuttersäuremethylester (HIBSM) enthalten. Die Menge an Carbonylverbindungen, insbesondere Aceton und / oder Acetaldehyd kann in der Reaktionsmischung in weiten Bereichen eingesetzt werden. Vorzugsweise wird die Carbonylverbindung in einer Menge im Bereich von 0,1-6 Mol, vorzugsweise 0,1-2 Mol pro Mol Cyanhydrin eingesetzt.

Die Temperatur, bei der Hydrolysereaktion erfolgt, kann im Allgemeinen im Bereich von 10-150°C, vorzugsweise im Bereich von 20-100°C und besonders bevorzugt im Bereich von 30-80°C liegen.

Die Reaktion kann beispielsweise in einem Festbettreaktor oder in einem Suspensionsreaktor durchgeführt werden.

Die so erhaltene Reaktionsmischung umfasst im Allgemeinen neben dem gewünschten Hydroxysäureamid weitere Bestandteile, insbesondere nicht umgesetztes Cyanhydrin sowie gegebenenfalls eingesetztes Aceton und /oder Acetaldehyd. Dementsprechend kann die Reaktionsmischung aufgereinigt werden, wobei nicht umgesetztes Cyanhydrin in Aceton und Blausäure gespalten werden kann, um diese wieder zur Herstellung des Cyanhydrins einzusetzen. Gleiches gilt für das abgetrennte Aceton und /oder Acetaldehyd.
Des Weiteren kann die aufgereinigte, Hydroxysäureamid umfassende Reaktionsmischung durch Ionenaustauschsäulen von weiteren Bestandteilen gereinigt werden.

Hierzu können insbesondere Kationenaustauscher und Anionenaustauscher eingesetzt werden. Hierfür geeignete Ionenaustauscher sind an sich bekannt. Beispielsweise können geeignete Kationenaustauscher durch Sulfonierung von Styrol-Divinylbenzolcopolymeren erhalten werden. Basische Anionenaustauscher umfassen quaternäre Ammoniumgruppen, die kovalent an Styrol-Divinylbenzolcopolymere gebunden sind.

Die Schritte zur Herstellung von α-Hydroxycarbonsäureamiden sind u.a. in EP-A-0686623 detaillierter beschrieben.

Im nächsten Schritt kann das so erhaltene α-Hydroxycarbonsäureamid zum α-Hydroxycarbonsäurealkylester umgesetzt werden. Dies kann beispielsweise durch die Verwendung von Alkylformiaten erfolgen. Insbesondere geeignet ist Methylformiat oder eine Mischung von Methanol und Kohlenmonooxid, wobei diese Reaktion beispielhaft in EP-A-0407811 beschrieben ist.

Bevorzugt erfolgt die Umsetzung des α-Hydroxycarbonsäureamids durch Alkoholyse mit einem Alkohol, der vorzugsweise 1-10 Kohlenstoffatome, besonders bevorzugt 1 bis 5 Kohlenstoffatome umfasst. Bevorzugte Alkohole sind u.a. Methanol, Ethanol, Propanol, Butanol, insbesondere n-Butanol und 2-Methyl-1-propanol, Pentanol, Hexanol, Heptanol, 2-Ethylhexanol, Octanol, Nonanol und Decanol. Besonders bevorzugt wird als Alkohol Methanol und / oder Ethanol eingesetzt, wobei Methanol ganz besonders bevorzugt ist. Die Umsetzung von Carbonsäureamiden mit Alkoholen, um Carbonsäureester zu erhalten, ist allgemein bekannt.

Diese Reaktion kann beispielsweise durch basische Katalysatoren beschleunigt werden. Diese umfassen homogene Katalysatoren sowie heterogene Katalysatoren.

Zu den homogenen Katalysatoren gehören Alkalimetallalkoholate und organometallische Verbindungen von Titan, Zinn und Aluminium. Vorzugsweise wird ein Titanalkoholat oder Zinnalkoholat, wie beispielsweise Titantetraisopropyloxid oder Zinntetrabutyloxid eingesetzt. Zu den heterogenen Katalysatoren gehören u.a. Magnesiumoxid, Calciumoxid sowie basische Ionenaustauscher, wie sie zuvor beschrieben wurden.

Das Molverhältnis von α-Hydroxycarbonsäureamid zu Alkohol, beispielsweise α-Hydroxyisobuttersäureamid zu Methanol, ist an sich nicht kritisch, wobei dieses vorzugsweise im Bereich von 2:1-1:20 liegt.

Die Reaktionstemperatur kann ebenfalls in weiten Bereichen liegen, wobei die Reaktionsgeschwindigkeit mit zunehmender Temperatur im Allgemeinen zunimmt. Die obere Temperaturgrenze ergibt sich im Allgemeinen aus dem Siedepunkt des eingesetzten Alkohols. Vorzugsweise liegt die Reaktionstemperatur im Bereich von 40-300°C, besonders bevorzugt 160-240°C. Die Reaktion kann, je nach Reaktionstemperatur, bei Unter oder Überdruck durchgeführt werden. Vorzugsweise wird diese Reaktion in einem Druckbereich von 0,5-35 bar, besonders bevorzugt 5 bis 30 bar durchgeführt.

Üblicherweise wird der entstehende Ammoniak aus dem Reaktionssystem abgeleitet, wobei die Umsetzung vielfach beim Siedepunkt durchgeführt wird.

Der bei der Alkoholyse freigesetzte Ammoniak kann dem Gesamtprozess auf leichte Weise zurück geführt werden. Beispielsweise kann Ammoniak mit Methanol zu Blausäure umgesetzt werden. Dies ist beispielsweise in EP-A-0941984 dargelegt. Des Weiteren kann die Blausäure aus Ammoniak und Methan gemäß dem BMA- oder Andrussow-Verfahren erhalten werden, wobei diese Verfahren in Ullmann's Encyclopedia of Industrial Chemistry 5. Auflage auf CD-ROM, Stichwort "Inorganic Cyano Compounds" beschrieben sind.

In einem nächsten Schritt wird der α-Hydroxycarbonsäurealkylester mit (Meth)acrylsäure umgesetzt, wobei Alkyl(meth)acrylat sowie α-Hydroxycarbonsäure erhalten werden.

Die Reaktionsmischung kann neben den Reaktanden weitere Bestandteile, wie beispielsweise Lösungsmittel, Katalysatoren, Polymerisationsinhibitoren und Wasser umfassen.

Die Umsetzung des Alkylhydroxycarbonsäureesters mit (Meth)acrylsäure kann durch mindestens eine Säure oder mindestens eine Base katalysiert werden. Hierbei können sowohl homogene als auch heterogene Katalysatoren verwendet werden. Als saure Katalysatoren besonders geeignet sind insbesondere anorganische Säuren, beispielsweise Schwefelsäure oder Salzsäure, sowie organische Säuren, beispielsweise Sulfonsäuren, insbesondere p-Toluolsulfonsäure sowie saure Kationenaustauscher.

Zu den besonders geeigneten Kationenaustauscherharzen gehören insbesondere sulfonsäuregruppenhaltige Styrol-Divinylbenzolpolymere. Besonders geeignete Kationenaustauscherharze können kommerziell von Rohm&Haas unter der Handelsbezeichnung Amberlyst® und von Bayer unter der Handelsbezeichnung Lewatit® erhalten werden.

Die Konzentration an Katalysator liegt vorzugsweise im Bereich von 1 bis 30 Gew.-%, besonders bevorzugt 5 bis 15 Gew.-%, bezogen auf die Summe des eingesetzten α-Alkylhydroxycarbonsäureesters und der eingesetzten (Meth)acrylsäure.

Zu den bevorzugt einsetzbaren Polymerisationsinhibitoren gehören unter anderem Phenothiazin, Tertiärbutylcatechol, Hydrochinonmonomethylether, Hydrochinon, 4-Hydroxy-2,2,6,6-tetramethylpiperidinooxyl (TEMPOL) oder deren Gemische; wobei die Wirksamkeit dieser Inhibitoren durch Einsatz von Sauerstoff teilweise verbessert werden kann. Die Polymerisationsinhibitoren können in einer Konzentration im Bereich von 0,001 bis 2,0 Gew.-%, besonders bevorzugt im Bereich von 0,01 bis 0,2 Gew.-%, bezogen auf die Summe des eingesetzten α-Alkylhydroxycarbonsäureesters und der eingesetzten (Meth)acrylsäure, eingesetzt werden.

Die Reaktion wird bevorzugt bei Temperaturen im Bereich von 50°C bis 200°C, besonders bevorzugt 70°C bis 130°C, insbesondere 80°C bis 120°C und ganz besonders bevorzugt 90°C bis 110°C durchgeführt.

Die Reaktion kann bei Unter- oder Überdruck durchgeführt werden, je nach Reaktionstemperatur. Vorzugsweise wird diese Reaktion beim Druckbereich von 0,02-5 bar, insbesondere 0,2 bis 3 bar und besonders bevorzugt 0,3 bis 0,5 bar durchgeführt.

Das molare Verhältnis von (Meth)acrylsäure zum α-Hydroxycarbonsäurealkylester liegt vorzugsweise im Bereich von 4:1-1:4, insbesondere 3:1 bis 1:3 und besonders bevorzugt im Bereich von 2:1-1:2.

Bevorzugt beträgt die Selektivität mindestens 90 %, besonders bevorzugt 98 %. Die Selektivität ist definiert als das Verhältnis der Summe aus gebildeten Stoffmengen an Alkyl(meth)acrylaten und α-Hydroxycarbonsäuren, bezogen auf die Summe der umgesetzten Stoffmengen an α-Hydroxycarbonsäurealkylester und (Meth)acrylsäure.

Gemäß einem besonderen Aspekt der vorliegenden Erfindung kann die Umesterung in Gegenwart von Wasser erfolgen. Vorzugsweise liegt der Wassergehalt im Bereich von 0,1-50 Gew.-%, besonders bevorzugt 0,5-20 Gew.-%, und ganz besonders bevorzugt 1-10 Gew.-%, bezogen auf das Gewicht des eingesetzten α-Hydroxycarbonsäurealkylesters.

Durch den Zusatz von geringen Mengen Wasser kann überraschend die Selektivität der Umsetzung erhöht werden. Trotz Wasserzugabe kann dabei die Bildung von Methanol überraschend gering gehalten werden.

Bei einer Wasserkonzentration von 10 bis 15 Gew.-%, bezogen auf das Gewicht des eingesetzten α-Hydroxycarbonsäurealkylesters, bilden sich vorzugsweise weniger als 5 Gew.-% Methanol bei einer Reaktionstemperatur von 120°C und einer Reaktionsdauer bzw. Verweilzeit von 5 bis 180 min.

Die Umesterung kann chargenweise oder kontinuierlich durchgeführt werden, wobei kontinuierliche Verfahren bevorzugt sind.

Die Reaktionsdauer der Umesterung hängt von den eingesetzten Molmassen sowie der Reaktionstemperatur ab, wobei dieser Parameter in weiten Bereichen liegen kann. Bevorzugt liegt die Reaktionszeit der Umesterung des α-Hydroxycarbonsäurealkylesters mit (Meth)acrylsäure im Bereich von 30 Sekunden bis 15 Stunden, besonders bevorzugt 5 Minuten bis 5 Stunden und ganz besonders bevorzugt 15 Minuten bis 3 Stunden.

Bei kontinuierlichen Verfahren beträgt die Verweilzeit vorzugsweise 30 Sekunden bis 15 Stunden, besonders bevorzugt 5 Minuten bis 5 Stunden und ganz besonders bevorzugt 15 Minuten bis 3 Stunden.

Bei der Herstellung von Methylmethacrylat aus α-Hydroxyisobuttersäuremethylester beträgt die Temperatur vorzugsweise 60 bis 130 °C, besonders bevorzugt 80 bis 120°C und ganz besonders bevorzugt 90 bis 110 °C. Der Druck liegt vorzugsweise im Bereich von 50 bis 1000 mbar, besonders bevorzugt 300 bis 800 mbar. Das molare Verhältnis von Methacrylsäure zu α-Hydroxyisobuttersäuremethylester liegt vorzugsweise im Bereich von 2:1-1:2, insbesondere 1,5:1-1:1,5.

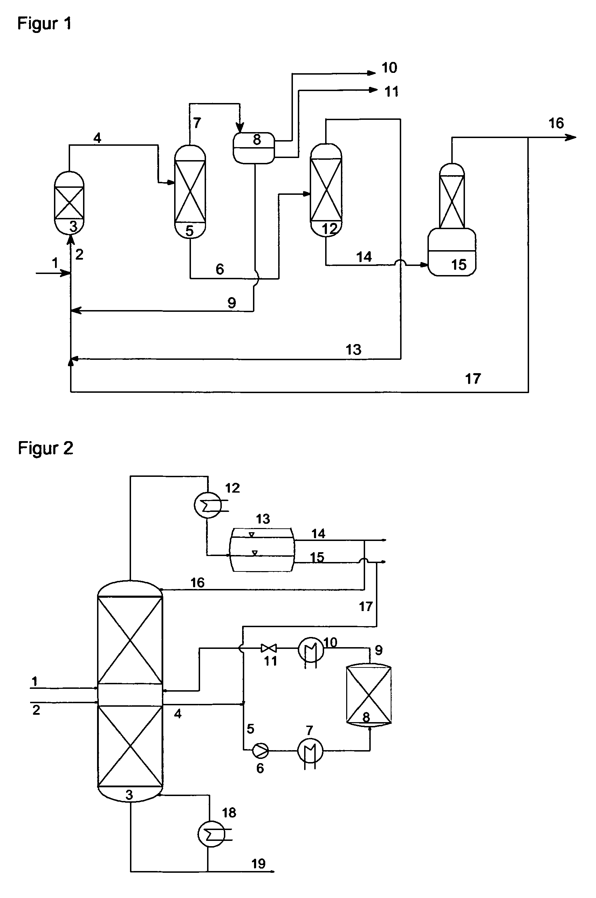

Beispielsweise kann die Umesterung in der in Fig. 1 dargelegten Anlage erfolgen. Der Hydroxycarbonsäureester, beispielsweise Hydroxyisobuttersäuremethylester, wird über Leitung (1) einem Festbettreaktor (3) zugeführt, der ein Kationenaustauschharz umfasst. (Meth)acrylsäure, beispielsweise 2-Methylpropensäure wird über Leitung (2) oder Leitung (17) in den Festbettreaktor (3) zugegegeben. Die Leitung (2) kann mit weiteren Leitungen, beispielsweise Leitung (9) und Leitung (13), verbunden sein, um so die Anzahl der Zuleitungen in den Reaktor zu verringern. Die Leitungen (9), (13) und/oder (17) können jedoch auch direkt in den Festbettreaktor führen. Bei den zuvor genannten Reaktionsbedingungen wird eine Reaktionsmischung gebildet, die neben Methanol sowie nicht umgesetztem Hydroxyisobuttersäuremethylester und Methacrylsäure die Reaktionsprodukte Hydroxyisobuttersäure sowie Methylmethacrylat umfasst. Diese Reaktionsmischung wird über Leitung (4) in eine Destille (5) geleitet. In der Destille (5) wird Wasser, Methylmethacrylat sowie Methanol als Destillat erhalten, welches über Leitung (7) als Kopfprodukt einem Phasentrenner (8) zugeführt wird. In der oberen Phase sammelt sich Methylmethacrylat sowie Methanol, welche über Leitung (10) aus dem System entnommen werden. In der unteren Phase des Phasentrenners (8) sammelt sich insbesondere Wasser, welches über Leitung (11) aus dem System entfernt oder über Leitung (9) dem Festbettreaktor (3) zugeführt werden kann.

Aus dem Sumpf können Hydroxyisobuttersäuremethylester, Hydroxyisobuttersäure sowie Methacrylsäure gewonnen werden, die über Leitung (6) in eine zweite Destille (12) geleitet werden können. Hierbei wird Hydroxyisobuttersäuremethylester sowie Methacrylsäure abdestilliert, die über Leitung (13) der Umesterung zurückgeführt werden. Die im Destillationssumpf enthaltene Hydroxyisobuttersäure wird über Leitung (14) in einen Reaktor zur Dehydratisierung (15) geleitet. Die hierdurch erhaltene Methacrylsäure kann über Leitung (17) der zuvor dargelegten Umesterung zugeführt oder über Leitung (16) dem System entnommen werden.

Gemäß einer besonders bevorzugten Ausführungsform kann die Umesterung in einer Destille erfolgen. Hierbei kann der Katalysator in jedem Bereich der Destille beigefügt werden. Beispielsweise kann der Katalysator in den Bereich des Sumpfes oder im Bereich der Kolonne bereitgestellt werden. Hierbei sollten die Edukte jedoch in Kontakt mit dem Katalysator gebracht werden. Des Weiteren kann Katalysator in einem separaten Bereich der Destille bereitgestellt werden, wobei dieser Bereich mit den weiteren Bereichen der Destille, beispielsweise des Sumpfes und/oder der Kolonne verbunden sind. Diese separate Anordnung des Katalysatorbereichs ist bevorzugt.

Durch diese bevorzugte Ausgestaltung gelingt es überraschend die Selektivität der Umsetzung zu erhöhen. In diesem Zusammenhang ist festzuhalten, dass der Druck der Umsetzung unabhängig vom Druck innerhalb der Destillationskolonnen eingestellt werden kann. Hierdurch kann die Siedetemperatur niedrig gehalten werden, ohne dass die Reaktionszeit bzw. die Verweilzeit entsprechend ansteigt. Darüber hinaus kann die Temperatur der Umsetzung über einen weiten Bereich variiert werden. Hierdurch kann die Reaktionszeit verkürzt werden. Darüber hinaus kann das Volumen an Katalysator beliebig gewählt werden, ohne dass auf die Geometrie der Kolonne Rücksicht genommen werden müsste. Weiterhin kann beispielsweise ein weiterer Reaktand hinzugefügt werden. All diese Maßnahmen können zur Steigerung der Selektivität und der Produktivität beitragen, wobei überraschende Synergieeffekte erzielt werden.

Hierbei wird der α-Hydroxycarbonsäurealkylester, beispielsweise α-Hydroxyisobuttersäuremethylester, der Destille zugeführt. Des Weiteren wird (Meth)acrylsäure, beispielsweise Methacrylsäure in die Destille eingeleitet. Die Destillationsbedingungen werden bevorzugt so ausgeführt, dass genau ein Produkt durch Destillation aus der Destille abgeleitet wird, wobei das zweite Produkt im Sumpf verbleibt und aus diesem kontinuierlich entfernt wird. Bei Verwendung von Alkoholen mit einer geringen Kohlenstoffanzahl, insbesondere Ethanol oder Methanol, wird vorzugsweise das Alkyl(meth)acrylat durch Destillation der Reaktionsmischung entzogen. Die Edukte werden zyklisch durch den Katalysatorbereich geleitet. Hierdurch entsteht kontinuierlich Alkyl(meth)acrylat sowie α-Hydroxycarbonsäure.

Eine bevorzugte Ausführungsform der Reaktivdestillation ist in Fig. 2 schematisch dargelegt. Die Edukte können über eine gemeinsame Leitung (1) oder getrennt über zwei Leitungen (1) und (2) in die Destillationskolonne (3) eingeleitet werden. Bevorzugt erfolgt die Zugabe der Edukte über getrennte Leitungen. Die Edukte können dabei auf derselben Stufe oder in beliebiger Position der Kolonne zugeführt werden.

Die Temperatur der Reaktanden kann dabei über einen Wärmetauscher in der Zuführung eingestellt werden, wobei die hierfür notwendigen Aggregate nicht in Figur 1 dargestellt sind. In einer bevorzugten Variante werden die Edukte separat in die Kolonne dosiert, wobei die Zudosierung der leichtersiedenden Komponente unterhalb der Position für die Zuführung der schwersiedenden Verbindung erfolgt. Bevorzugt wird in diesem Fall die leichtersiedende Komponente dampfförmig zugegeben.

Für die vorliegende Erfindung kann jede mehrstufige Destillationskolonne (3) verwendet werden, die zwei oder mehr Trennstufen besitzt. Als Anzahl der Trennstufen wird in der vorliegenden Erfindung die Anzahl der Böden bei einer Bodenkolonne oder die Anzahl der theoretischen Trennstufen im Fall einer Packungskolonne oder eine Kolonne mit Füllkörpern bezeichnet.

Beispiele für eine mehrstufige Destillationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine mehrstufige Destillationskolonne mit Füllkörpern solche wie Raschig-Ringe, Lessing-Ringe, Pall-Ringe, Berl-Sättel, Intalox Sättel und für eine mehrstufige Destillationskolonne mit Packungen wie solche vom Typ Mellapak (Sulzer), Rombopak (Kühni), Montz-Pak (Montz) und Packungen mit Katalysatortaschen, beispielsweise Kata-Pak.

Eine Destillationskolonne mit Kombinationen aus Bereichen von Böden, aus Bereichen von Füllkörpern oder aus Bereichen von Packungen kann ebenso verwendet werden.

Die Kolonne (3) kann mit Einbauten ausgestattet sein. Die Kolonne weist vorzugsweise einen Kondensator (12) zur Kondensation des Dampfes und einen Sumpf-Verdampfer (18) auf.

Vorzugsweise weist die Destillationsapparatur mindestens einen Bereich, nachfolgend Reaktor genannt, auf, in dem mindestens ein Katalysator vorgesehen ist. Dieser Reaktor kann innerhalb der Destillationskolonne liegen. Vorzugsweise wird dieser Reaktor jedoch außerhalb der Kolonne (3) in einem separaten Bereich angeordnet, wobei eine dieser bevorzugten Ausführungsformen in Figur 2 näher erläutert wird.

Um die Umesterungsreaktion in einem separaten Reaktor (8) durchzuführen, kann innerhalb der Kolonne ein Teil der abwärts strömenden flüssigen Phase über einen Sammler aufgefangen und als Teilstrom (4) aus der Kolonne geleitet. Die Position des Sammlers wird durch das Konzentrationsprofil in der Kolonne der einzelnen Komponenten bestimmt. Das Konzentrationsprofil kann hierbei über die Temperatur und/oder den Rücklauf geregelt werden. Der Sammler wird bevorzugt so positioniert, dass der aus der Kolonne herausgeführte Strom beide Reaktanden, besonders bevorzugt die Reaktanden in ausreichend hoher Konzentration und ganz besonders bevorzugt im molaren Verhältnis Säure : Ester = 1,5:1 bis 1:1,5 enthält. Des Weiteren können mehrere Sammler an verschiedenen Stellen der Destillationskolonne vorgesehen sein, wobei durch die Menge an entnommenen Reaktanden die molaren Verhältnisse eingestellt werden können.

Dem aus der Kolonne abgeführten Strom kann zudem noch ein weiterer Reaktand, beispielsweise Wasser zudosiert werden, um das Produktverhältnis Säure/Ester in der Kreuzumesterungsreaktion einzustellen oder die Selektivität zu erhöhen. Das Wasser kann über eine Leitung von außen zugeführt (in Figur 1 nicht dargestellt) oder aus einem Phasentrenner (13) entnommen werden. Der Druck des mit Wasser angereicherten Stroms (5) kann anschließend über ein Mittel zur Druckerhöhung (6), beispielsweise eine Pumpe, erhöht werden.

Durch eine Erhöhung des Drucks kann eine Bildung von Dampf in dem Reaktor, beispielsweise einem Festbettreaktor vermindert bzw. verhindert werden. Hierdurch kann eine gleichmäßige Durchströmung des Reaktors und Benetzung der Katalysatorpartikel erzielt werden. Der Strom kann durch einen Wärmetauscher (7) geführt und die Reaktionstemperatur eingestellt werden. Der Strom kann dabei je nach Bedarf erwärmt oder gekühlt werden. Über die Reaktionstemperatur kann zudem das Produktverhältnis Ester zu Säure eingestellt werden.

Im Festbettreaktor (8) findet am Katalysator die Umesterungsreaktion statt. Der Reaktor kann dabei abwärts oder aufwärts durchströmt werden. Der die zu einem gewissen Anteil die Produkte und die nicht umgesetzten Edukte enthaltende Reaktorabstrom (9), wobei der Anteil der Komponenten im Reaktorabstrom von der Verweilzeit, der Katalysatormasse, der Reaktionstemperatur und dem Eduktverhältnis sowie der zugesetzten Wassermenge abhängt, wird zunächst durch einen Wärmetauscher (10) geleitet und auf eine Temperatur eingestellt, die beim Einleiten in die Destillationskolonne vorteilhaft ist. Bevorzugt wird die Temperatur eingestellt, die der Temperatur in der Destillationskolonne an der Stelle des Einleitens des Stroms entspricht.

Die Position, wo der den Reaktor verlassende Strom in die Kolonne zurückgeführt wird, kann dabei ober- oder unterhalb der Position für die Entnahme der Reaktorzuführung liegen, bevorzugt wird jedoch oberhalb. Vor dem Rückführen in die Kolonne kann der Strom über ein Ventil (11) entspannt werden, wobei vorzugsweise das gleiche Druckniveau wie in der Kolonne eingestellt wird. Bevorzugt weist die Destillationskolonne hierbei einen niedrigeren Druck auf. Diese Ausgestaltung bietet den Vorteil, dass die Siedepunkte der zu trennenden Komponenten herabgesetzt werden, wodurch die Destillation auf niedrigerem Temperaturniveau, dadurch energiesparender und thermisch schonender durchgeführt werden kann.

In der Destillationskolonne (3) erfolgt dann die Auftrennung des Produktgemisches. Der Niedersieder, bevorzugt der in der Umesterung gebildete Ester, wird über Kopf abgetrennt. Bevorzugt wird die Destillationskolonne so gefahren, dass das vor dem Festbettreaktor zugegebene Wasser ebenfalls als Kopfprodukt abgetrennt wird. Der am Kopf abgezogene, dampfförmige Strom wird in einem Kondensator (12) kondensiert und anschließend in einem Dekanter (13) in die wässrige und Produktesterhaltige Phase aufgetrennt. Die wässrige Phase kann über eine Leitung (15) zur Aufarbeitung ausgeschleust oder vollständig oder teilweise über Leitung (17) als Strom wieder in die Reaktion zurückgeführt werden. Der Strom aus der esterhaltigen Phase kann über Leitung (14) zum Teil als Rücklauf (16) auf die Kolonne gefahren werden oder zum Teil aus der Destille ausgeschleust werden. Der Schwersieder, bevorzugt die in der Kreuzumesterung gebildete Säure, wird als Sumpfstrom aus der Kolonne (19) ausgeschleust.

Durch diese bevorzugte Ausgestaltung gelingt es überraschend die Selektivität der Umsetzung zu erhöhen. In diesem Zusammenhang ist festzuhalten, dass der Druck der Umsetzung unabhängig vom Druck innerhalb der Destillationskolonnen eingestellt werden kann. Hierdurch kann die Siedetemperatur niedrig gehalten werden, ohne dass die Reaktionszeit bzw. die Verweilzeit entsprechend ansteigt. Darüber hinaus kann die Temperatur der Umsetzung über einen weiten Bereich variiert werden. Hierdurch kann die Reaktionszeit verkürzt werden. Darüber hinaus kann das Volumen an Katalysator beliebig gewählt werden, ohne dass auf die Geometrie der Kolonne Rücksicht genommen werden müsste. Weiterhin kann beispielsweise ein weiterer Reaktand hinzugegeben werden.

Die aus der Reaktion erhaltene α-Hydroxycarbonsäure, beispielsweise Hydroxyisobuttersäure, kann auf bekannte Weise dehydratisiert werden. Im Allgemeinen wird die α-Hydroxycarbonsäure, beispielsweise die α-Hydroxyisobuttersäure in Gegenwart mindestens eines Metallsalzes, beispielsweise von Alkali- und/oder Erdalkalimetallsalzen, auf Temperaturen im Bereich von 160-300°C, besonders bevorzugt im Bereich von 200 bis 240 °C erhitzt, wobei im Allgemeinen die (Meth)acrylsäure sowie Wasser erhalten werden. Zu den geeigneten Metallsalzen gehören u.a. Natriumhydroxid, Kaliumhydroxid, Calciumhydroxid, Bariumhydroxid, Magnesiumhydroxid, Natriumsulfit, Natriumcarbonat, Kaliumcarbonat, Strontiumcarbonat, Magnesiumcarbonat, Natriumbicarbonat, Natriumacetat, Kaliumacetat und Natriumdihydrogenphosphat.

Die Dehydratisierung der α-Hydroxycarbonsäure kann vorzugsweise bei einem Druck im Bereich von 0,05 bar bis 2,5 bar, besonders bevorzugt im Bereich von 0,1 bar bis 1 bar durchgeführt werden.

Gemäß einem besonderen Aspekt der vorliegenden Erfindung ist der Druck bei der Dehydratisierung in etwa gleich groß wie der Druck bei der zuvor beschriebenen Umesterung des α-Hydroxycarbonsäurealkylesters mit der (Meth)acrylsäure, ohne dass hierdurch eine Beschränkung erfolgen soll. Vorzugsweise ist die Differenz des Druckes bei der Umesterung und der Dehydratisierung kleiner als 0,1 bar, besonders bevorzugt kleiner als 0,05 bar. Gemäß einer besonderen Ausführungsform der vorliegenden Erfindung kann die gasförmig erhaltene (Meth)acrylsäure ohne Kondensation und erneutes Verdampfen zur Umesterung geleitet werden.

Die Dehydratisierung von α-Hydroxycarbonsäuren ist beispielsweise in DE-A-176 82 53 beschrieben.

Die so erhaltene (Meth)acrylsäure kann wiederum zur Herstellung von Alkyl(meth)acrylaten eingesetzt werden. Des Weiteren ist (Meth)acrylsäure ein Handelsprodukt. Überraschend kann dementsprechend die Anlage zur Herstellung von Alkyl(meth)acrylaten ebenfalls dazu dienen, (Meth)acrylsäure herzustellen, wobei das Produktverhältnis von Alkyl(meth)acrylaten zu (Meth)acrylsäure durch die Konzentration an Wasser bei der Umesterung des α-Hydroxycarbonsäurealkylesters und/oder durch die Reaktionstemperatur leicht reguliert werden kann.

Insgesamt können somit aus Carbonylverbindungen, Blausäure und Alkoholen Alkyl(meth)acrylate auf einfache und kostengünstige Weise durch Verfahren erhalten werden, die die folgenden Schritte umfassen:
A) Bildung mindestens eines Cyanhydrins durch Umsetzung mindestens einer Carbonylverbindung mit Blausäure;
B) Hydrolyse des Cyanhydrins bzw. der Cyanhydrine unter Bildung mindestens eines α-Hydroxycarbonsäureamids;
C) Alkoholyse des α-Hydroxycarbonsäureamids bzw. der α-Hydroxycarbonsäureamide, wobei mindestens ein α-Hydroxycarbonsäurealkylester erhalten wird;
D) Umesterung des α-Hydroxycarbonsäurealkylesters bzw. der α-Hydroxycarbonsäurealkylester mit (Meth)acrylsäure, wobei mindestens ein Alkyl(meth)acrylat und mindestens eine α-Hydroxycarbonsäure gebildet werden;
E) Dehydratisierung der α-Hydroxycarbonsäure bzw. der α-Hydroxycarbonsäuren, wobei (Meth)acrylsäure gebildet wird.

Nachfolgend soll die vorliegende Erfindung anhand von Beispielen sowie eines Vergleichsbeispiels näher erläutert werden.

### Beispiel 1

In einer in Fig. 2 dargelegten Reaktivdestille wurden über einen Zeitraum von 48 Stunden 4619 g α-Hydroxyisobuttersäuremethylester (HIBSM) sowie 3516 g Methacrylsäure (MAS) zugeführt. Die Reaktion wurde bei einer Temperatur von 120°C und einem Druck von 250 mbar durchgeführt. Entstandene α-Hydroxyisobuttersäure wurde aus dem Sumpf entfernt. Methylmethacrylat (MMA) wurde abdestilliert. Die Reaktion wurde in Gegenwart von 16 Gew.-% Wasser, bezogen auf das Gewicht an α-Hydroxyisobuttersäuremethylester, durchgeführt. Die Umsetzung wurde unter Verwendung eines sauren Katalysators durchgeführt (Kationenaustauscher; Lewatit®-Typ K2431 von Bayer).

Die als das Verhältnis von gebildeten Stoffmengen an Methylmethacrylat (MMA) und α-Hydroxyisobuttersäure (HIBS) zu umgesetzten Stoffmengen an HIBSM und MAS definierte Selektivität betrug 99%

Die aus dem Verfahren gewonnene α-Hydroxyisobuttersäure wurde gemäß DE-OS 17 68 253 dehydratisiert.

Insgesamt ergibt sich eine Selektivität von 98,5%, die als das Verhältnis von gebildeter Stoffmenge an MMA zur umgesetzten Stoffmenge an HIBSM definiert ist.

### Vergleichsbeispiel 1

Methylmethacrylat wurde hergestellt durch Dehydratisierung von α-Hydroxyisobuttersäuremethylester. Diese Reaktion wurde gemäß EP-A-0941984 durchgeführt. Eine Mischung von 20 g Natriumdihydrogenphosphat und 80 g Wasser wurden zu 60 g Silikagel zugegeben. Das Wasser wurde aus der Mischung unter einem reduzierten Druck entfernt. Der Rückstand wurde über Nacht bei 150°C getrocknet, um einen Katalysator zu erhalten. 10 g des erhaltenen Katalysators wurden in eine Quarzröhre gegeben, der mit einem Verdampfer ausgestattet war. Die Quarzröhre wurde mit einem Ofen erhitzt, wobei die Temperatur der Katalysatorschicht etwa 400°C betrug. Eine Mischung von Methanol und α-Hydroxyisobuttersäuremethylester (2:1) wurden kontinuierlich mit einer Geschwindigkeit von 10 g pro Stunde verdampft und über die Katalysatorschicht geleitet. Die als das Verhältnis von gebildeter Stoffmenge an MMA zur umgesetzten Stoffmenge an HIBSM definierte Selektivität der Umsetzung betrug 88 %.

### Beispiele 2 bis 18

Das Beispiel 1 wurde im Wesentlichen wiederholt, wobei jedoch der Reaktionsmischung kein Wasser hinzugegeben wurde. Die Umsetzung erfolgte unter den in der Tabelle 1 angegebenen Bedingungen, insbesondere hinsichtlich der Temperatur, Verweilzeit und molarem Verhältnis der Edukte. Die als das Verhältnis von gebildeten Stoffmengen an MMA und HIBS zu umgesetzten Stoffmengen an HIBSM und MAS definierte Selektivität der Umsetzungen ist ebenfalls in Tabelle 1 dargelegt.

**Tabelle 1**

| Beispiel | Reaktionstemperatur [°C] | Molares Verhältnis HIBSM/MAS | Verweilzeit [min] | Selektivität [%] |
|---|---|---|---|---|
| 2 | 120 | 1,00 | 28,33 | 93,21 |
| 3 | 90 | 1,00 | 42,50 | 95,06 |
| 4 | 100 | 1,00 | 42,50 | 94,81 |
| 5 | 110 | 1,00 | 42,50 | 94,64 |
| 6 | 120 | 1,00 | 42,50 | 90,67 |
| 7 | 90 | 1,00 | 85,00 | 95,53 |
| 8 | 100 | 1,00 | 85,00 | 94,95 |
| 9 | 110 | 1,00 | 85,00 | 93,55 |
| 10 | 120 | 1,00 | 85,00 | 91,78 |
| 11 | 90 | 1,00 | 170,00 | 94,83 |
| 12 | 100 | 1,00 | 170,00 | 94,06 |
| 13 | 90 | 2,0 | 42,50 | 91,61 |
| 14 | 100 | 2,0 | 42,50 | 91,73 |
| 15 | 90 | 2,0 | 85,00 | 90,63 |
| 16 | 100 | 2,0 | 85,00 | 90,30 |
| 17 | 120 | 0,50 | 28,33 | 92,05 |
| 18 | 120 | 0,50 | 42,50 | 92,62 |

### Beispiele 19 bis 38

Das Beispiel 1 wurde im Wesentlichen wiederholt, wobei jedoch die Umsetzung unter den in der Tabelle 2 angegebenen Bedingungen, insbesondere hinsichtlich der Temperatur und Verweilzeit durchgeführt wurden. Das molare Verhältnis von HIBSM/MAS betrug 1:1. Des Weiteren wurden unterschiedliche Anteile an Wasser zugegeben, wobei diese ebenfalls in Tabelle 2 dargelegt sind. Die als das Verhältnis von gebildeten Stoffmengen an MMA und HIBS zu umgesetzten Stoffmengen an HIBSM und MAS definierte Selektivität der Umsetzungen sowie das molare Verhältnis an HIBS zu MMA sind ebenfalls in Tabelle 2 aufgeführt.

**Tabelle 2**

| Bsp. | Reaktionstemperatur [°C] | Molares Verhältnis H₂O zu HIBSM | Verweilzeit [min] | Selektivität [%] | Molares Verhältnis HIBS zu MMA |
|---|---|---|---|---|---|
| 19 | 90 | 0,20 | 42,5 | 98,61 | 1,33 |
| 20 | 100 | 0,20 | 42,5 | 98,18 | 1,21 |
| 21 | 110 | 0,20 | 42,5 | 97,44 | 1,11 |
| 22 | 120 | 0,20 | 42,5 | 96,27 | 0,99 |
| 23 | 90 | 0,20 | 85 | 98,34 | 1,18 |
| 24 | 100 | 0,20 | 85 | 97,66 | 1,09 |
| 25 | 110 | 0,20 | 85 | 96,56 | 1,02 |
| 26 | 100 | 0,20 | 170 | 96,95 | 1,00 |
| 27 | 90 | 0,50 | 42,5 | 98,80 | 1,61 |
| 28 | 100 | 0,50 | 42,5 | 98,64 | 1,36 |
| 29 | 110 | 0,50 | 42,5 | 98,21 | 1,22 |
| 30 | 120 | 0,50 | 42,5 | 97,58 | 1,08 |
| 31 | 90 | 0,50 | 85 | 98,76 | 1,39 |
| 32 | 100 | 0,50 | 85 | 98,35 | 1,20 |
| 33 | 110 | 0,50 | 85 | 97,78 | 1,10 |
| 34 | 100 | 0,50 | 170 | 98,08 | 1,10 |
| 35 | 90 | 1,00 | 50,0 | 99,41 | 2,090 |
| 36 | 100 | 1,00 | 50,0 | 99,65 | 1,618 |
| 37 | 110 | 1,00 | 50,0 | 99,82 | 1,360 |
| 38 | 120 | 1,00 | 50,0 | 99,54 | 1,319 |

Die obigen Beispiele zeigen, dass durch die vorliegenden Erfindung Alkyl(meth)acrylate mit sehr hoher Selektivität gebildet werden kann, wobei das Verhältnis von Alkyl(meth)acrylate zu α-Hydroxycarbonsäure auch bei relativ hohen Wasserkonzentrationen nahe 1 ist. Dementsprechend wird relativ wenig Methanol gebildet. Das molare Verhältnis von Alkyl(meth)acrylate zu α-Hydroxycarbonsäure kann hierbei auch über die Temperatur gesteuert werden.

## Patentansprüche

1. Verfahren zur Herstellung von Alkyl(meth)acrylaten, umfassend die Schritte
Umesterung eines α-Hydroxycarbonsäurealkylesters mit (Meth)acrylsäure, wobei Alkyl(meth)acrylate sowie α-Hydroxycarbonsäure erhalten werden, und
Dehydratisierung der α-Hydroxycarbonsäure, wobei (Meth)acrylsäure erhalten wird.

2. Verfahren gemäß Anspruch 1, **dadurch gekennzeichnet, dass** der α-Hydroxycarbonsäurealkylester durch Alkoholyse eines Hydroxycarbonsäuresäureamids erhalten wird.

3. Verfahren gemäß Anspruch 2, **dadurch gekennzeichnet, dass** das Hydroxycarbonsäuresäureamid durch Hydrolyse eines Cyanhydrins erhalten wird.

4. Verfahren gemäß Anspruch 3, **dadurch gekennzeichnet, dass** das Cyanhydrin Acetoncyanhydrin ist.

5. Verfahren gemäß Anspruch 3 oder 4, **dadurch gekennzeichnet, dass** zur Hydrolyse ein Katalysator eingesetzt wird.

6. Verfahren gemäß Anspruch 5, **dadurch gekennzeichnet, dass** der Katalysator Manganoxid, Schwefelsäure oder ein Enzym umfasst.

7. Verfahren gemäß mindestens einem der vorhergehenden Ansprüche 2 bis 6, **dadurch gekennzeichnet, dass** der zur Alkoholyse des Hydroxycarbonsäuresäureamids eingesetzte Alkohol 1 bis 10 Kohlenstoffatome umfasst.

8. Verfahren gemäß Anspruch 7, **dadurch gekennzeichnet, dass** der Alkohol Methanol und/oder Ethanol ist.

9. Verfahren gemäß mindestens einem der vorhergehenden Ansprüche 2 bis 8, **dadurch gekennzeichnet, dass** die Alkoholyse bei einer Temperatur im Bereich von 160-240°C durchgeführt wird.

10. Verfahren gemäß mindestens einem der vorhergehenden Ansprüche 2 bis 9, **dadurch gekennzeichnet, dass** die Alkoholyse bei einem Druck im Bereich von 5 bis 30 bar durchgeführt wird.

11. Verfahren gemäß mindestens einem der vorhergehenden Ansprüche 2 bis 10, **dadurch gekennzeichnet, dass** zur Alkoholyse mindestens ein basischer Katalysator eingesetzt wird.

12. Verfahren gemäß mindestens einem der vorhergehenden Ansprüche, **dadurch gekennzeichnet, dass** die Umesterung des α-Hydroxycarbonsäurealkylesters mit (Meth)acrylsäure durch eine Säure katalysiert wird.

13. Verfahren gemäß Anspruch 12, **dadurch gekennzeichnet, dass** die Säure ein Ionenaustauscher ist.

14. Verfahren gemäß Anspruch 12 oder 13, **dadurch gekennzeichnet, dass** die Umesterung in einer Destille durchgeführt wird.

15. Verfahren gemäß mindestens einem der vorhergehenden Ansprüche, **dadurch gekennzeichnet, dass** die Umesterung des α-Hydroxycarbonsäurealkylesters mit (Meth)acrylsäure bei einem Druck im Bereich von 100 mbar bis 3 bar durchgeführt wird.

16. Verfahren gemäß mindestens einem der vorhergehenden Ansprüche, **dadurch gekennzeichnet, dass** die Umesterung des α-Hydroxycarbonsäurealkylesters mit (Meth)acrylsäure bei einer Temperatur im Bereich von 70 bis 130°C durchgeführt wird.

17. Verfahren gemäß mindestens einem der vorhergehenden Ansprüche, **dadurch gekennzeichnet, dass** die Umesterung des α-Hydroxycarbonsäurealkylesters mit (Meth)acrylsäure in Gegenwart von Wasser durchgeführt wird.

18. Verfahren gemäß Anspruch 17, **dadurch gekennzeichnet, dass** die Wasserkonzentration 0,1 bis 50 Gew.-%, bezogen auf das Gewicht des α-Hydroxycarbonsäurealkylesters beträgt.

19. Verfahren gemäß mindestens einem der vorhergehenden Ansprüche, **dadurch gekennzeichnet, dass** das molare Verhältnis von α-Hydroxycarbonsäurealkylester zu (Meth)acrylsäure bei der Umesterung des α-Hydroxycarbonsäurealkylesters mit (Meth)acrylsäure im Bereich von 3:1 bis 1:3 liegt.

20. Verfahren gemäß mindestens einem der vorhergehenden Ansprüche, **dadurch gekennzeichnet, dass** die Reaktionszeit bei der Umesterung des a-Hydroxycarbonsäurealkylesters mit (Meth)acrylsäure im Bereich von 5 Minuten bis 5 Stunden liegt.

21. Verfahren gemäß mindestens einem der vorhergehenden Ansprüche, **dadurch gekennzeichnet, dass** die Dehydratisierung der α-Hydroxycarbonsäure und die Umesterung des α-Hydroxycarbonsäurealkylesters mit (Meth)acrylsäure bei gleichem Druck durchgeführt wird.

22. Verfahren gemäß mindestens einem der vorhergehenden Ansprüche, **dadurch gekennzeichnet, dass** die durch die Dehydratisierung der α-Hydroxycarbonsäure gasförmig erhaltene (Meth)acrylsäure ohne Kondensation und erneutes Verdampfen zur Umesterung geleitet wird.

## Claims

1. Process for preparing alkyl (meth)acrylates, comprising the steps of transesterifying an alkyl α-hydroxycarboxylate with (meth)acrylic acid to obtain alkyl (meth)acrylates and α-hydroxycarboxylic acid, and dehydrating the α-hydroxycarboxylic acid to obtain (meth)acrylic acid.

2. Process according to Claim 1, **characterized in that** the alkyl α-hydroxycarboxylate is obtained by alcoholysis of a hydroxycarboxamide.

3. Process according to Claim 2, **characterized in that** the hydroxycarboxamide is obtained by a hydrolysis of a cyanohydrin.

4. Process according to Claim 3, **characterized in that** the cyanohydrin is acetone cyanohydrin.

5. Process according to Claim 3 or 4, **characterized in that** a catalyst is used for the hydrolysis.

6. Process according to Claim 5, **characterized in that** the catalyst comprises manganese oxide, sulphuric acid or an enzyme.

7. Process according to at least one of the preceding Claims 2 to 6, **characterized in that** the alcohol used for the alcoholysis of the hydroxycarboxamide comprises 1 to 10 carbon atoms.

8. Process according to Claim 7, **characterized in that** the alcohol is methanol and/or ethanol.

9. Process according to at least one of the preceding Claims 2 to 8, **characterized in that** the alcoholysis is carried out at a temperature in the range of 160-240°C.

10. Process according to at least one of the preceding Claims 2 to 9, **characterized in that** the alcoholysis is carried out at a pressure in the range from 5 to 30 bar.

11. Process according to at least one of the preceding Claims 2 to 10, **characterized in that** at least one basic catalyst is used for the alcoholysis.

12. Process according to at least one of the preceding claims, **characterized in that** the transesterification of the alkyl α-hydroxycarboxylate with (meth)acrylic acid is catalysed by an acid.

13. Process according to Claim 12, **characterized in that** the acid is an ion exchanger.

14. Process according to Claim 12 or 13, **characterized in that** the transesterification is carried out in a still.

15. Process according to at least one of the preceding claims, **characterized in that** the transesterification of the alkyl α-hydroxycarboxylate with (meth)acrylic acid is carried out at a pressure in the range from 100 mbar to 3 bar.

16. Process according to at least one of the preceding claims, **characterized in that** the transesterification of the alkyl α-hydroxycarboxylate with (meth)acrylic acid is carried out at a temperature in the range from 70 to 130°C.

17. Process according to at least one of the preceding claims, **characterized in that** the transesterification of the alkyl α-hydroxycarboxylate with (meth)acrylic acid is carried out in the presence of water.

18. Process according to Claim 17, **characterized in that** the water concentration is 0.1 to 50% by weight based on the weight of the alkyl α-hydroxycarboxylate.

19. Process according to at least one of the preceding claims, **characterized in that** the molar ratio of alkyl α-hydroxycarboxylate to (meth)acrylic acid in the transesterification of the alkyl α-hydroxycarboxylate with (meth)acrylic acid is in the range from 3:1 to 1:3.

20. Process according to at least one of the preceding claims, **characterized in that** the reaction time in the transesterification of the alkyl α-hydroxycarboxylate with (meth)acrylic acid is in the range from 5 minutes to 5 hours.

21. Process according to at least one of the preceding claims, **characterized in that** the dehydration of the a-hydroxycarboxylic acid and the transesterification of the alkyl α-hydroxycarboxylate with (meth)acrylic acid is carried out at the same pressure.

22. Process according to at least one of the preceding claims, **characterized in that** the (meth)acrylic acid obtained in gaseous form by the dehydration of the α-hydroxycarboxylic acid is passed to the transesterification without condensation and reevaporation.

## Revendications

1. Procédé de fabrication de (méth)acrylates d'alkyle, comprenant les étapes suivantes :
la transestérification d'un ester alkylique de l'acide α-hydroxycarboxylique avec de l'acide (méth)acrylique, des (méth)acrylates d'alkyle et de l'acide α-hydroxycarboxylique étant obtenus, et
la déshydratation de l'acide α-hydroxycarboxylique, de l'acide (méth)acrylique étant obtenu.

2. Procédé selon la revendication 1, **caractérisé en ce que** l'ester alkylique de l'acide α-hydroxycarboxylique est obtenu par alcoolyse d'un amide de l'acide hydroxycarboxylique.

3. Procédé selon la revendication 2, **caractérisé en ce que** l'amide de l'acide hydroxycarboxylique est obtenu par hydrolyse d'une cyanhydrine.

4. Procédé selon la revendication 3, **caractérisé en ce que** la cyanhydrine est l'acétone-cyanhydrine.

5. Procédé selon la revendication 3 ou 4, **caractérisé en ce qu'**un catalyseur est utilisé pour l'hydrolyse.

6. Procédé selon la revendication 5, **caractérisé en ce que** le catalyseur comprend de l'oxyde de manganèse, de l'acide sulfurique ou une enzyme.

7. Procédé selon au moins l'une quelconque des revendications 2 à 6 précédentes, **caractérisé en ce que** l'alcool utilisé pour l'alcoolyse de l'amide de l'acide hydroxycarboxylique comprend 1 à 10 atomes de carbone.

8. Procédé selon la revendication 7, **caractérisé en ce que** l'alcool est le méthanol et/ou l'éthanol.

9. Procédé selon au moins l'une quelconque des revendications 2 à 8 précédentes, **caractérisé en ce que** l'alcoolyse est réalisée à une température dans la plage allant de 160 à 240 °C.

10. Procédé selon au moins l'une quelconque des revendications 2 à 9 précédentes, **caractérisé en ce que** l'alcoolyse est réalisée à une pression dans la plage allant de 5 à 30 bar.

11. Procédé selon au moins l'une quelconque des revendications 2 à 10 précédentes, **caractérisé en ce qu'**au moins un catalyseur basique est utilisé pour l'alcoolyse.

12. Procédé selon au moins l'une quelconque des revendications précédentes, **caractérisé en ce que** la transestérification de l'ester alkylique de l'acide α-hydroxycarboxylique avec de l'acide (méth)acrylique est catalysée par un acide.

13. Procédé selon la revendication 12, **caractérisé en ce que** l'acide est un échangeur d'ions.

14. Procédé selon la revendication 12 ou 13, **caractérisé en ce que** la transestérification est réalisée dans une colonne de distillation.

15. Procédé selon au moins l'une quelconque des revendications précédentes, **caractérisé en ce que** la transestérification de l'ester alkylique de l'acide α-hydroxycarboxylique avec de l'acide (méth)acrylique est réalisée à une pression dans la plage allant de 100 mbar à 3 bar.

16. Procédé selon au moins l'une quelconque des revendications précédentes, **caractérisé en ce que** la transestérification de l'ester alkylique de l'acide α-hydroxycarboxylique avec de l'acide (méth)acrylique est réalisée à une température dans la plage allant de 70 à 130 °C.

17. Procédé selon au moins l'une quelconque des revendications précédentes, **caractérisé en ce que** la transestérification de l'ester alkylique de l'acide α-hydroxycarboxylique avec de l'acide (méth)acrylique est réalisée en présence d'eau.

18. Procédé selon la revendication 17, **caractérisé en ce que** la concentration en eau est de 0,1 à 50 % en poids, par rapport au poids de l'ester alkylique de l'acide α-hydroxycarboxylique.

19. Procédé selon au moins l'une quelconque des revendications précédentes, **caractérisé en ce que** le rapport molaire entre l'ester alkylique de l'acide α-hydroxycarboxylique et l'acide (méth)acrylique lors de la transestérification de l'ester alkylique de l'acide α-hydroxycarboxylique avec de l'acide (méth)acrylique se situe dans la plage allant de 3:1 à 1:3.

20. Procédé selon au moins l'une quelconque des revendications précédentes, **caractérisé en ce que** la durée de réaction lors de la transestérification de l'ester alkylique de l'acide α-hydroxycarboxylique avec de l'acide (méth)acrylique se situe dans la plage allant de 5 minutes à 5 heures.

21. Procédé selon au moins l'une quelconque des revendications précédentes, **caractérisé en ce que** la déshydratation de l'acide α-hydroxycarboxylique et la transestérification de l'ester alkylique de l'acide α-hydroxycarboxylique avec de l'acide (méth)acrylique sont réalisées à la même pression.

22. Procédé selon au moins l'une quelconque des revendications précédentes, **caractérisé en ce que** l'acide (méth)acrylique obtenu sous forme gazeuse par la déshydratation de l'acide α-hydroxycarboxylique est conduit sans condensation et nouvelle évaporation dans la transestérification.
